# EUROPÄISCHE PATENTSCHRIFT

(11) **EP 2 498 897 B1**
(45) Veröffentlichungstag und Bekanntmachung des Hinweises auf die Patenterteilung: **14.09.2016**
(21) Anmeldenummer: 10781588.8
(22) Anmeldetag: 24.09.2010
(51) Int. Cl.: B01D 53/18, B01D 53/78, C07D 251/60, F28D 9/00, F28F 3/14, F28F 13/06

(54) **GASWÄSCHER MIT INTEGRIERTEM WÄRMETAUSCHER**
GAS SCRUBBER HAVING AN INTEGRATED HEAT EXCHANGER
LAVEUR DE GAZ AVEC ÉCHANGEUR DE CHALEUR INTÉGRÉ

(30) Priorität: 11.11.2009 DE 102009052506
(43) Veröffentlichungstag der Anmeldung: 19.09.2012
(73) Patentinhaber: Air Liquide Global E&C Solutions Germany GmbH, 60439 Frankfurt am Main (DE)
(72) Erfinder: CASTILLO-WELTER, Frank, 61381 Friedrichsdorf (DE); STEDEN, Christoph, 61440 Oberursel (DE); WALTER, Dominic, 64289 Darmstadt (DE); EHRING, Georg, 60316 Frankfurt (DE); MÜLLER-HASKY, Martin, 63150 Heusenstamm (DE)
(86) Internationale Anmeldenummer: PCT/DE2010/001136
(87) Internationale Veröffentlichungsnummer: WO 2011/057597

(56) Entgegenhaltungen:
- EP-A1- 1 138 676
- EP-A2- 0 862 036
- WO-A1-96/07467
- DE-A1- 4 300 131
- GB-A- 1 147 908
- US-A- 5 603 377
- US-A1- 2005 020 851

## Beschreibung

Die Erfindung betrifft einen Gaswäscher, mit einem oder mehreren, im Apparategehäuse integrierten, Wärmetauschern, zur Kühlung des aus dem zu reinigenden Gas und der Waschflüssigkeit gebildeten Gas/Flüssig-Gemischs, wobei der oder die Wärmetauscher aus Thermoblechen bestehen und wobei der Apparat die Form eines senkrecht stehenden Zylinders hat und wobei der oder die Wärmetauscher jeweils den gesamten Querschnitt des Apparats ausfüllen, wobei die Thermobleche senkrecht stehend angeordnet sind und wobei das Gas/Flüssig-Gemisch durch die Spalten zwischen den Thermoblechen und die Kühlflüssigkeit innerhalb der Thermobleche strömt.

Die Erfindung betrifft außerdem ein Verfahren zur Verwendung eines solchen Gaswäschers.

Gaswäscher und Verfahren zur Kühlung und Reinigung sind bekannt.

In besonderen Anwendungsfällen für Gaswäscher, in denen die Temperatur des zu kühlenden Gases weit über der der Waschflüssigkeit liegt und gleichzeitig eine Erwärmung der Waschflüssigkeit soweit wie möglich verhindert werden soll, um deren Verdampfung oder chemische Zersetzung zu vermeiden, ist es erforderlich Wärmetauscher direkt im Wäschergehäuse, in der Strömung des Gas/Flüssig-Gemischs, zu integrieren.

In der deutschen Offenlegungsschrift 25 25 781 wird ein entsprechender Fall zur Behandlung von Prozessabgas aus einer Melaminsynthese beschrieben. Dabei wird ein Gaswäscher mit einem integrierten Kühler verwendet, um zu verhindern, dass der als Waschflüssigkeit verwendete Harnstoff durch das mit hoher Temperatur in den Wäscher gelangende Prozessabgas zu stark erwärmt wird und dadurch gebildete Zersetzungsprodukte des Harnstoffs die Wärmetauscheroberfläche verschmutzen.

Der Ullmann's Encyclopedia of Industrial Chemistry, Sixth Edition, 1998 Electronic Release kann man entnehmen, dass für die gleiche Anwendung, ein im wesentlichen zylindrischer, aufrecht stehender Gaswäscher mit integriertem Wärmetauscher verwendet wird, der vom Gas und von der Waschflüssigkeit von oben nach unten, im Gleichstrom, durchlaufen wird.

Über die Bauart des oder der im Wäscher integrierten Wärmetauscher wird in beiden Schriften nichts ausgesagt.

Dem Fachmann ist aber bekannt, dass für die in den vorgenannten Schriften beschriebenen Anwendungen Rohrbündelwärmetauscher eingesetzt werden, und zwar sowohl in der Art, dass das zu kühlende Gas/Flüssig-Gemisch um die Rohre, als auch, dass es durch die Rohre geführt wird.

Wenn das Gas/Flüssig-Gemisch um die Rohre geführt wird, werden die Rohre vertikal, und damit quer zur Strömungsrichtung, im Gaswäscher installiert. Dabei ist es möglich, die Rohre quer durch den Wäscher, von Wand zu Wand, zu verlegen oder U-Rohrbündelwärmetauscher vertikal durch die Kolonnenwand in den Wäscher einzuführen. Die Bauart der Rohrführung von Wand zu Wand hat den Nachteil, dass der Wärmetauscher für Reinigungs- und Reparaturarbeiten nur sehr schwer zugänglich ist, auch ist diese Bauart sehr kostenaufwendig. Die Wäscherbauart, bei der U-Rohrbündelwärmetauscher seitlich in den Wäscherquerschnitt geschoben werden ist preisgünstiger und wartungsfreundlicher. Sie hat aber den Nachteil, dass die Wärmetauscherfläche nur ungleichmäßig über den Wäscherquerschnitt verteilt ist, sodass das Gas-Flüssiggemisch nur ungleichmäßig gekühlt wird. Bei der Bauart, bei der das Gas-Flüssiggemisch durch die Wärmetauscherrohre geführt wird, verlaufen die Rohre parallel zur senkrechten Achse des Wäschers und die Rohrplatten füllen den gesamten Querschnitt des Wäschers aus. Der Nachteil dieser Bauart liegt in der Neigung der Rohre zu Verstopfen und damit in hohem Reinigungsaufwand.

In der britischen Anmeldeschrift 1,147,908 wird eine Absorptionskolonne vorgestellt, in der ein Gas mit einer Flüssigkeit in Kontakt gebracht werden soll. Um die dabei entstehende Absorptions- und Reaktionswärme abzuführen, soll der Kontakt auf der Fläche eines Wärmeaustauschers erfolgen. Dafür wird an
Stelle der bekannten Rohrbündel die Verwendung von plattenförmigen Wärmetauscherflächen empfohlen, da diese, bei gleicher Wärmeaustauschfläche einen geringeren Raumbedarf haben.

Die europäische Anmeldeschrift EP 0 862 036 A2 zeigt einen Apparat zur Kühlung eines Gas/Flüssig-Stroms der mit Plattenwärmetauschern ausgestattet ist. Um den Kontakt der zu kühlenden Flüssigkeit mit der Wärmetauscherfläche zu verbessern, wird hier vorgeschlagen, die Fläche mit einem Drahtnetz zu überziehen. Nachteilig dabei ist, dass eine derartige Konstruktion die Bildung von Ablagerungen auf der Wärmetauscherfläche begünstigt und außerdem schwer zu reinigen ist. Es bestand die Aufgabe, einen verbesserten, auch zur Kühlung der Prozessmedien geeigneten, Gaswäscher zu entwerfen, mit dem eine gleichmäßigere Kühlung möglich ist und der mit geringem Reinigungs- und Wartungsaufwand betrieben werden kann.

Die Aufgabe wird in der vorliegenden Erfindung durch die Verwendung eines Gaswäschers gemäß den Merkmalen des Anspruchs 1 gelöst. Weiterbildungen, Vorteile und Anwendungsmöglichkeiten der Erfindung ergeben sich auch aus der nachfolgenden Beschreibung und der Zeichnung.

Thermobleche und aus diesen aufgebaute Wärmetauscher sind seit den 1970-ziger Jahren bekannt (US 3,822,742; W. Mühlthaler, Fachhochschule Mannheim, AA Nr. 13). Thermobleche werden aus zwei gleichstarken, aufeinander gelegten und durch eine umlaufende Naht am Rand verschweißte Bleche gebildet. Auf der Fläche sind die Bleche durch regelmäßig angeordnete Punktschweißungen verbunden. Durch Erzeugung eines hydraulischen oder pneumatischen Drucks zwischen den Blechen werden diese zu der charakteristischen Steppkissenform der Thermobleche geformt. Der so erhaltene Raum zwischen den Blechen dient zur Aufnahme des Wärmeträger- oder Kühlmittels. Da in diesem Raum keine Strömungswege vorgegeben sind, eignen sich Thermobleche besonders zur Verdampfungskühlung, da die entstehenden Dampfblasen ungehindert aufsteigen und das Thermoblech verlassen können.

Erfindungsgemäß werden zahlreiche Thermobleche zu einem Wärmetauscher zusammengefasst und sind senkrecht stehend, parallel zur senkrechten Achse im zylindrischen Gaswäscher integriert, wobei der gesamte Querschnitt des Gaswäschers ausgefüllt wird und, gemäß einer bevorzugten Ausführungsform der Erfindung, ein freier Abstand von Blech zu Blech von 7 bis 45 mm, bevorzugt von 15 bis 30 mm, besteht.

Durch Verwendung dieser Wärmetauscherbauart steht eine gleichmäßig über den Querschnitt des Gaswäschers angeordnete spezifische Dichte an Wärmetauscherfläche zur Verfügung.

Es ist auch möglich, aus Kostengründen, derartige Wärmetauscher mit quadratischem oder rechteckigem Querschnitt in den kreisförmigen Querschnitt des Wäschers einzubauen. Die dabei freibleibenden Kreissegmente um den Wärmetauscher müssen dann mit Hilfe von entsprechenden Leitblechen abgedeckt werden, um ungekühlte Randströmungen zu vermeiden.

Ein Vorteil von aus Thermoblechen aufgebauten Wärmetauschern ist es, dass sie, im Verhältnis zur von ihnen gelieferten Wärmetauscherfläche, nur einen geringen Druckverlust bei der Durchströmung des Gas-Flüssiggemisches verursachen. Ein wichtiger Vorteil dieser Bauart ist die Fähigkeit zur Selbstreinigung des Wärmetauschers bei dieser Verfahrensweise, denn sollten sich aus der Melaminsynthese stammende Partikelklumpen zwischen den Thermoblechen festsetzen, so bleibt genügend Raum für die Gas-Flüssigströmung einen solchen Klumpen zu umströmen und abzutragen. Bei einer Strömung durch Wärmetauscherrohre, würde dagegen ein solcher Klumpen zur Verstopfung des Rohres führen, ohne dass die Möglichkeit zur Selbstreinigung besteht.

Daher besteht ein weiteres Merkmal der Erfindung darin, dass die Waschflüssigkeit und das Prozessgas gemeinsam durch die Spalten zwischen den Thermoblechen strömt und das Kühlmedium innerhalb der Thermobleche strömt.

Weiterhin ist die Erfindung dadurch gekennzeichnet, dass stromwärts vor dem Wärmetauscher Strömungsleitbleche montiert sind, wobei diese aus jeweils einer rechteckigen Blechplatte bestehen, die parallel zueinander angeordnet sind, wobei die Bleche so angeordnet sind, dass deren untere, lange Kanten parallel, in einem Abstand von 5 bis 15 cm, zu den oberen Kanten der Thermobleche verlaufen, wobei die unteren Kanten der Leitbleche den gleichen Abstand zueinander haben, wie die Thermobleche,
wobei die kurzen Kanten der Leitbleche eine gleiche Länge von 10 bis 30 cm haben, und wobei die Leitbleche zur senkrechten Achse des Gaswäschers in einem Winkel von kleiner als 90° angeordnet sind. Die Aufgabe dieser Leitbleche ist es, die Strömung des Gas-Flüssiggemisches gegen die Thermobleche zu lenken, um den Wärmeaustausch weiter zu verbessern.

Eine weitere bevorzugte Ausgestaltung der Erfindung ist dadurch gekennzeichnet, dass die Strömungsleitbleche in nebeneinander liegenden und dabei parallel zueinander verlaufenden Reihen, mit alternierenden Winkeln F, angeordnet sind.

Um die Turbulenz der Strömung des zu kühlenden Gas/Flüssig-Gemischs und damit die Wärmeübertragung zu erhöhen, werden in einer weiteren Ausgestaltung der Erfindung die Thermobleche auf einander folgender Wärmetauscher gegeneinander um einen Winkel von 90° versetzt angeordnet. Die erfindungsgemäße Bauart eines Gaswäschers ist insbesondere dazu geeignet das Prozessabgas aus einer Melaminsynthese zu kühlen und zu reinigen, die in der Gasphase und bei Drücken von höchstens 10 bar durchgeführt wird. Hierdurch ist das sogenannte BASF Verfahren charakterisiert, wie es in Ullmann's Encyclopedia of Industrial Chemistry, Sixth Edition, 1998 Electronic Release beschrieben ist.

Eine weitere bevorzugte Ausgestaltung der Erfindung ist dadurch gekennzeichnet, dass das innerhalb der Thermobleche befindliche Kühlmedium, durch die Aufnahme der Wärme aus dem Gasflüssiggemisch, verdampft wird. Auf diese Weise kann die Temperatur der Wärmetauscheroberfläche genau und gleichmäßig eingestellt werden.

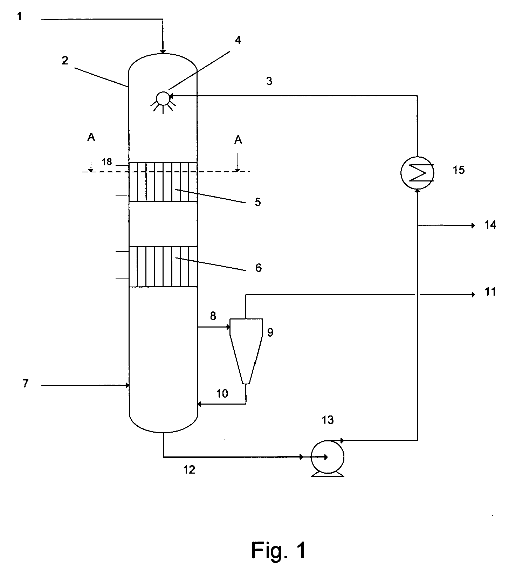

Anhand des Beispiels 1, mit der Stoffstromtabelle 1 und der Zeichnung, bestehend aus Fig. 1 bis 3, soll das Verfahren erläutert werden.

### Beispiel 1:

In Fig. 1 ist eine Anlage zur Wäsche eines hauptsächlich aus Ammoniak und Kohlendioxid, sowie Harnstoff- und Isocyansäureresten bestehenden Prozessabgases einer Melaminsynthese mit dem Gaswäscher (2), dem Abscheider (9), der Kreislaufpumpe (13) und dem Kühler dargestellt.

Der Prozessabgasstrom (1) tritt, aus der, nicht dargestellten, Melaminsynthese kommend, am Kopf des zylindrischen Gaswäschers (2) ein. Als Stoffstrom (3)
wird die, als Waschflüssigkeit verwendete, harnstoffhaltige Schmelze in den Gaswäscher eingeführt. Mittels eines Düsensystems (4) wird die Waschflüssigkeit (3) im Prozessabgas (1) verteilt, sodass ein Gas/FlüssigGemisch gebildet wird. Anschließend durchströmt das Gas/Flüssig-Gemisch die Wärmetauscher (5) und (6). Die Wärmetauscher sind erfindungsgemäß aus Thermoblechen aufgebaut. In den Thermoblechen befindet sich, zur Kühlung des Gas/Flüssig-Gemisches, siedendes Wasser mit einer Temperatur von 125°C. Der durch die aufgenommene Wärme erzeugte Wasserdampf verlässt die Thermobleche über Sammelleitung (18), wird kondensiert (nicht dargestellt), woraufhin das Kondensat dem Wärmetauscher wieder zugeführt wird. Nach dem Durchströmen der Wärmetauscher (5) und (6) werden das Prozessabgas- und die Waschflüssigkeit von einander getrennt. Dazu wird das Gas-Flüssiggemisch über Leitung (8) aus dem Wäscher in einen Gas-Flüssig-Abscheider (9) geleitet. Über Leitung (11) verlässt das Gas den Abscheider (9) und kann als Prozessgas in der Melaminsynthese verwendet werden. Über Leitung (10) wird die harnstoffhaltige Schmelze aus dem Gas-Flüssig-Abscheider (9) zurück in den Sumpf des Gaswäschers (2) geleitet, von wo aus sie über Leitung (12), Kreislaufpumpe (13) und Wärmetauscher (15) als Stoffstrom (3) wieder in den Wäscher (2) geleitet wird. Wärmetauscher (15) dient zur Feineinstellung der - Temperatur der Waschflüssigkeit. Über Leitung (7) wird frische Harnstoffschmelze in den Wäscher (2) eingespeist und vermischt sich dort mit der als Waschflüssigkeit verwendeten harnstoffhaltigen Schmelze. Aus dem Kreislauf der harnstoffhaltigen Schmelze wird Stoffstrom (14) abgezweigt und zur Verwendung als Rohstoff in die (nicht dargestellte) Melaminsynthese geleitet.

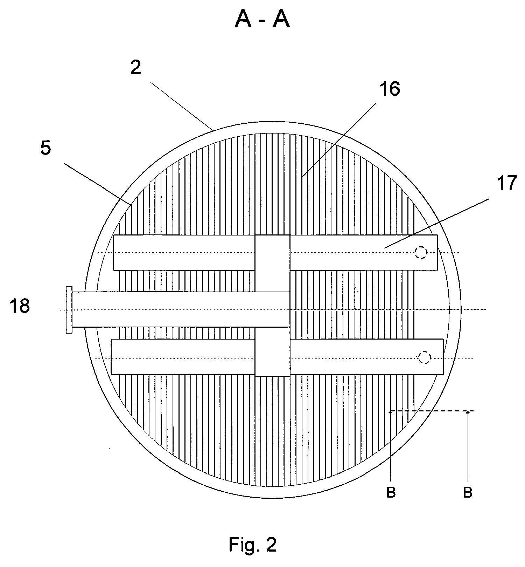

Fig. 2 zeigt den Querschnitt A-A. Dargestellt ist der Wärmetauscher (5) innerhalb des Wäschers (2), mit den parallel zu einander stehenden Thermoblechen (16), den Sammelleitungen (17) und der Anschlussleitung (18) für das Kühlmedium.

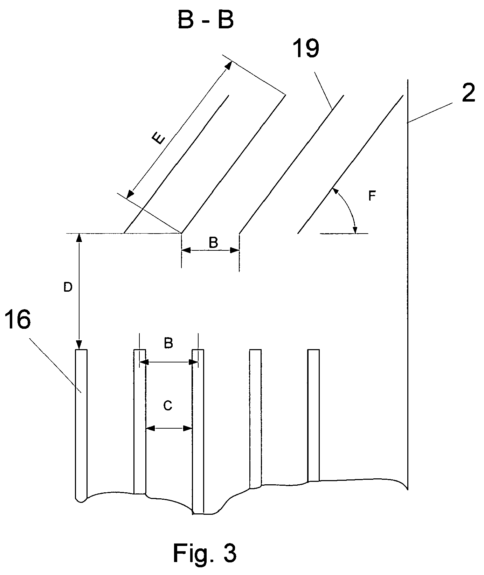

Fig. 3 zeigt den Längsschnitt B-B. Dargestellt ist ein Ausschnitt der Wand des Wäschers (2) und ein Ausschnitt des Wärmetauschers (5) mit dessen Thermoblechen (16). Diese sind mit einem Mittenabstand B und einem freien Zwischenabstand C parallel zueinander angeordnet. Im Abstand D über den Thermoblechen (16) sind Strömungsleitbleche (19) der Länge E installiert. Der horizontale Abstand zwischen den Strömungsleitblechen ist gleich groß wie der Mittenabstand der Thermobleche.

**Stoffstromtabelle 1:**

| Stoffstrom-Nr. | | 1 | 3 | 7 | 8 | 10 | 11 | 12 | 14 |
|---|---|---|---|---|---|---|---|---|---|
| Harnstoff | | Reste | x | x | x | x | | x | x |
| Prozessgas (NH₃ + CO₂) | | x | | | x | | x | | |
| HNCO, Reste | | x | | | | | | | |
| Melamin, Reste | | x | x | | x | x | | x | x |
| Durchfluss | t/h | 150,0 | 854,0 | 10,0 | 1003,9 | 855,6 | 148,3 | 865,6 | 11,7 |
| Temp. | °C | 231 | 131 | 138 | 138 | 138 | 138 | 138 | 138 |
| Druck | bar | 2,7 | | | | | 2,6 | | |
| Aggregatzustand | *) | g | fl | fl | g,fl | fl | g | fl | fl |

| | | | | | | | | | |
|---|---|---|---|---|---|---|---|---|---|
| *) fl = flüssig g = gasförmig | | | | | | | | | |

## Patentansprüche

1. Gaswäscher, mit einem oder mehreren, im Apparategehäuse integrierten, Wärmetauschern, zur Kühlung des aus dem zu reinigenden Gas und der Waschflüssigkeit gebildeten Gas/Flüssig-Gemischs, wobei der oder die Wärmetauscher aus Thermoblechen bestehen und wobei der Apparat die Form eines senkrecht stehenden Zylinders hat und wobei der oder die Wärmetauscher jeweils den gesamten Querschnitt des Apparats ausfüllen, wobei die Thermobleche senkrecht stehend angeordnet sind und wobei das Gas/Flüssig-Gemisch durch die Spalten zwischen den Thermoblechen und die Kühlflüssigkeit innerhalb der Thermobleche strömt **dadurch gekennzeichnet, dass** stromwärts vor dem obersten Wärmetauscher Strömungsleitbleche (19) montiert sind, wobei es sich bei diesen um rechteckige Blechplatten handelt, die parallel zueinander und
so angeordnet sind, dass deren untere Kanten parallel, in einem Abstand D von 5 bis 15 cm, zu den oberen Kanten der Thermobleche (16) verlaufen, wobei der waagerechte Mittenabstand (B) der Leitbleche gleich dem Mittenabstand der Thermobleche ist, wobei die Tiefe E der Leitbleche zwischen 10 bis 30 cm beträgt, und wobei die Leitbleche zur senkrechten Achse des Gaswäschers in einem Winkel F von kleiner als 90° angeordnet sind.

2. Gaswäscher nach Anspruch 1, **dadurch gekennzeichnet, dass** der freie Abstand C zwischen den Thermoblechen 7 bis 45 mm, bevorzugt 15 bis 30 mm, beträgt.

3. Gaswäscher nach Anspruch 1 oder 2, **dadurch gekennzeichnet, dass** die Strömungsleitbleche in nebeneinander liegenden und dabei parallel zueinander verlaufenden Reihen, mit alternierenden Winkeln F, angeordnet sind.

4. Gaswäscher nach einem der vorhergehenden Ansprüche, **dadurch gekennzeichnet, dass** die Thermobleche stromwärts hintereinander eingebauter Wärmetauscher in einem Winkel von 90°, um die senkrechte Achse des Gaswäschers verdreht, zueinander angeordnet sind .

5. Verfahren zur Wäsche und Kühlung eines Gases unter Verwendung eines Gaswäschers nach den Ansprüchen 1 bis 4.

6. Verfahren gemäß Anspruch 5 zur Wäsche und Kühlung eines aus Ammoniak und Kohlendioxid, sowie Harnstoff- und Isocyansäureresten bestehenden Prozessabgases einer Melaminsynthese.

7. Verfahren gemäß Anspruch 5, **dadurch gekennzeichnet, dass** die innerhalb der Thermobleche befindliche Kühlflüssigkeit, durch die Aufnahme der Wärme aus dem Gas/Flüssig-Gemisch, verdampft wird.

## Claims

1. A gas scrubber, comprising one or more heat exchangers integrated in the apparatus housing for cooling the gas/liquid mixture formed of the gas to be cleaned and the washing liquid, wherein the heat exchanger or heat exchangers consist of thermoplates and wherein the apparatus has the shape of an upright cylinder and wherein the heat exchanger or heat exchangers each fill up the entire cross-section of the apparatus, wherein the thermoplates are arranged upright and wherein the gas/liquid mixture flows through the gaps between the thermoplates and the cooling liquid within the thermoplates, **characterized in that** flow baffle plates (19) are mounted in flow direction before the uppermost heat exchanger, wherein the same are rectangular sheet metal plates which are arranged parallel to each other and in such a way that their lower edges extend parallel to the upper edges of the thermoplates (16) at a distance D of 5 to 15 cm, wherein the horizontal center distance (B) of the baffle plates is equal to the center distance of the thermoplates, wherein the depth E of the baffle plates is between 10 and 30 cm, and wherein the baffle plates are arranged at an angle F of less than 90° with respect to the vertical axis of the gas scrubber.

2. The gas scrubber according to claim 1, **characterized in that** the free distance C between the thermoplates amounts to 7 to 45 mm, preferably 15 to 30 mm.

3. The gas scrubber according to claim 1 or 2, **characterized in that** the flow baffle plates are arranged in rows lying one beside the other and extending parallel to each other, with alternating angles F.

4. The gas scrubber according to any of the preceding claims, **characterized in that** the thermoplates of heat exchangers mounted one behind the other in flow direction are arranged at an angle of 90° relative to each other, rotated about the vertical axis of the gas scrubber.

5. A method for washing and cooling a gas by using a gas scrubber according to claims 1 to 4.

6. The method according to claim 5 for washing and cooling a process waste gas of a melamine synthesis, which consists of ammonia and carbon dioxide as well as urea and isocyanic acid residues.

7. The method according to claim 5, **characterized in that** the cooling liquid present within the thermoplates is evaporated by the absorption of heat from the gas/liquid mixture.

## Revendications

1. Laveur de gaz comprenant un ou plusieurs échangeurs de chaleur intégrés dans l'enveloppe de l'appareil pour le refroidissement du mélange gaz/liquide, formé du gaz à épurer et du liquide de lavage, le ou les échangeurs de chaleur étant en tôles thermiques et l'appareil ayant la forme d'un cylindre se tenant verticalement et le ou les échangeurs de chaleur remplissant respectivement toute la section transversale de l'appareil, les tôles thermiques étant disposées en se tenant verticalement, et le mélange gaz/liquide passant dans les intervalles entre les tôles thermiques et le liquide de refroidissement à l'intérieur des tôles thermiques, **caractérisé en ce qu'**en amont de l'échangeur de chaleur le plus haut, sont montés des chicanes (19) qui sont des plaques de tôle rectangulaires, disposées parallèlement entre elles et de façon telle que leurs bords inférieurs s'étendent parallèlement à une distance (D) de 5 à 15 cm des bords supérieurs des tôles (16) thermiques, la distance (B) moyenne horizontale entre les chicanes étant égale à la distance moyenne entre les tôles thermiques, la profondeur E des chicanes va de 10 à 30 cm et les chicanes faisant, avec l'axe vertical du laveur de gaz, un angle F plus petit que 90°.

2. Laveur de gaz suivant la revendication 1, **caractérisé en ce que** la distance C libre entre les tôles thermiques va de 7 à 45 mm, de préférence de 15 à 30 mm.

3. Laveur de gaz suivant la revendication 1 ou 2, **caractérisé en ce que** les chicanes sont disposées en rangées se trouvant les unes à côté des autres et s'étendant parallèlement entre elles, avec des angles F en alternance.

4. Laveur de gaz suivant l'une des revendications précédentes, **caractérisé en ce que** les échangeurs de chaleur intégrés en tôles thermiques sont disposés les uns derrière les autres dans le sens du courant, suivant un angle de 90 ° , en tournant autour de l'axe vertical du laveur de gaz.

5. Procédé de lavage et de refroidissement d'un gaz en utilisant un laveur de gaz suivant les revendications 1 à 4.

6. Procédé suivant la revendication 5 de lavage et de refroidissement d'un gaz de processus d'une synthèse de mélamine constitué d'ammoniac et de dioxyde de carbone ainsi que d'ester d'urée et d'ester d'acide isocyanique.

7. Procédé suivant la revendication 5, **caractérisé en ce que** l'on évapore le liquide de refroidissement se trouvant à l'intérieur des tôles thermiques par l'absorption de la chaleur provenant du mélange gaz-liquide.
